(19) **Europäisches Patentamt / European Patent Office / Office européen des brevets**

(11) **EP 4 252 549 A1**

(12) **EUROPEAN PATENT APPLICATION**

(43) Date of publication:
04.10.2023 Bulletin 2023/40

(51) International Patent Classification (IPC):
*A23L 13/00* (2016.01)  *A23L 17/00* (2016.01)
*C12N 5/00* (2006.01)  *C12N 5/077* (2010.01)
*A23J 3/22* (2006.01)

(21) Application number: 22164793.6

(22) Date of filing: 28.03.2022

(52) Cooperative Patent Classification (CPC):
A23L 13/00; A23J 3/227; A23J 3/26; A23L 17/00;
C12N 5/0068; C12N 5/0658; D01D 5/06;
D01D 5/12; D01F 9/04

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Mirai Foods AG
8820 Wädenswil (CH)

(72) Inventors:
• RAZZA, Nicolo
8804 Au ZH (CH)
• MOREL, Alexander
4600 Olten (CH)
• MUNAFO, Sara
8004 Zürich (CH)
• DAS, Suman Kumar
8800 Thalwil (CH)

(74) Representative: Bremi, Tobias Hans
Isler & Pedrazzini AG
Giesshübelstrasse 45
Postfach 1772
8027 Zürich (CH)

(54) **METHODS AND COMPOSITIONS FOR THE PREPARATION OF FIBROUS MUSCLE BUNDLES FOR CULTIVATED MEAT PRODUCTION**

(57) Paste for the fabrication of cultivated meat, composed of the following components:
(A) at least one polysaccharide crosslinkable to form a gel in a concentration in the paste in the range of 0.01-200 g per L of component (D);
(B) at least one protein assembling with the polysaccharide of component (A) via supramolecular or covalent interaction or a combination thereof, in a concentration in the paste in the range of 0.001-500 g per L of component (D);
(C) cells, in particular selected from the group consisting of embryonic stem cells, adult stem cells, induced stem cells, fibroblasts, fibrocytes or a combination thereof, in a concentration in the paste in the range of 0-300 billion cells per L of component (D);
(D) water or a water-based culturing medium;
(E) additives different from (A)-(D)
and methods for forming fibre starting from such a paste by adding a micro-structuring agent and extrusion drawing from a nozzle ready for conversion into muscle cells by culturing in differentiation media designed for the used cell types.

In the examples bicarbonate as microstructuring agent is disclosed as essential element which achieves protein unfolding and a spongy texture of the resulting hydrogel due to liberation of CO2.

FIG. 4

EP 4 252 549 A1

**Description**

TECHNICAL FIELD

The present invention relates to methods and compositions for the in-vitro preparation of muscle tissue for cultured meat production allowing the scalable production of muscle bundles of high quality and in large quantities. More specifically, the invention relates to (i) compositions for a gel paste which is turned within minutes into highly anisotropic fibrous gel embedding cells, (ii) a method for the fabrication of fibrous cell-laden gels, and (iii) compositions of the crosslinking baths used in the process. The fibrillar microstructure of the gel supports cell differentiation into muscle bundle with fibrillar and highly anisotropic texture. Also products obtained using such methods are the object of the invention.

PRIOR ART

Meat is an important protein source of the human diet. Controversial animal welfare associated with the traditional meat industry along with the increasing global population and demand for meat products, sustainable production alternatives are indispensable. Cultivated meat technology provides an opportunity to produce edible sources of animal protein that are not associated with the environmental impact of animal farming. In meat products, myofibrillar proteins are responsible for texture, tenderness, organoleptic and nutritional characteristics of meat products. In its current status, cultivated meat production is challenged by the availability of processing aids and components safe for human consumption as well as scalable and inexpensive processes and compositions to produce meat with high anisotropic cellular structuration characteristic of animal muscle tissue.

In order to replicate a fibrillar structuration in cultured meat product, it is important to employ scaffolding technique that guides the muscle progenitor stem cells to maturely differentiate in anisotropic muscle structure. Like meat obtained from the flesh of slaughtered animals, cultivated meat products should be produced in very large quantities and to a reasonably low price. For these reasons, not only the scaffolding technique employed are expected to be safe for human and non-human consumption, but they should also be scalable and compatible with high production rates. Keeping these points in mind, prior art available in patents and scientific literature is discussed in the following paragraphs.

In WO-A-2020160533 fibrous scaffolds for cultivated meat production are disclosed. The scaffold consists of a porous fibrous mat. Cells are integrated in the product by seeding the scaffold with a cell suspension. The seeding step is the critical one, and it can last hours to days depending on the used cells. Furthermore, it requires the design of specific seeding tanks in cultivated meat production plants. The seeding efficiency is controlled by the cell attachment kinetic and by the bioavailability of attachment sites. Low seeding efficiency can be experienced. The seeding is limited by the available surface area, and consequently, the cell density achievable in the final products is limited.

In MacQueen a al. (Muscle tissue engineering in fibrous gelatin: implications for meat analogs. npj Sci Food 3, 20 (2019)) the preparation of fibrous gelatin scaffold is reported by immersion rotary jet spinning at high rates (~ 100 g/h, dry weight) and, depending on process conditions, the approach allows the fabrication of edible scaffolds for cultured meat production in a scalable manner. However, as the scaffold relies on post-production cell seeding, the achievable seeding density is limited by the surface available area as confirmed by the lack of a mature muscle architecture.

In US7,622,299, bioengineered tissue substitutes are reported. The invention reports the fabrication of muscle tissues with a high degree of anisotropy consisting of aligned microfibers array. In this invention, microfibers need to be prepared individually and then arranged in arrays consisting of multiple fibers in parallel stacked in layers. Additionally, cells need to be seeded on the construct. Therefore, the same drawbacks mentioned for other porous-scaffolds seeded post-fabrication will also apply here.

In Kang et al (Engineered whole cut meat-like tissue by the assembly of cell fibers using tendon-gel integrated bioprinting, NATURE COMMUNICATIONS | (2021) 12:5059), a scaffolding method based on cell-laden gels is reported. The reported method overcome some of the seeding issues mentioned for the above-mentioned prior art. The gel formulation is based on fibrinogen crosslinked by thrombin and embedding cell and collagen microfibers. Because of the composition of such formulation, the sol to gel transition has a slow kinetics and therefore to produce fibers by molding in a sacrificial gel. Because of these reasons, the disclosed method and compositions are hardly scalable to produce a large quantity of cultivated meat.

US8,273,373 discloses a gel paste formulation allowing good degradability and bioactivity in combination with good scalability (due to the fast gelation allowed by the photocrosslinkable polysaccharide). The proposed cell in formulation could allow the fabrication of muscle constructs with high cell seeding efficiency (cell loaded can be loaded in the gel paste). Nevertheless, the fabrication of anisotropic muscle constructs mimicking the structural element of meat texture (fibrillar texture) is not possible. Moreover, the chemical modifications applied to the biopolymer are not approved for human consumption are their preparation is not cost-efficient for cultivated meat production.

WO 2021/158105 discloses a specific molecular weight and backbone structure to prepare alginate-based hydrogels with improved degradability and bioactivity, in some embodiments modified with bioactive peptides. The disclosed formulations allow the preparation of gel pastes embedding cells which can be crosslinked quickly by using Ca2+ ions. The proposed cell formulation could allow the fabrication of muscle constructs with high cell seeding efficiency (cell loaded can be loaded in the gel paste). Nevertheless, the lack of structuring agent and supramolecular interactions between hydrogel components does not allow the generation of anisotropic fibrous texture, and it turn, it is not possible to produce anisotropic muscle constructs mimicking the structural element of meat texture.

In Distler et al (3D printed oxidized alginate-gelatin bioink provides guidance for C2C12 muscle precursor cell orientation and differentiation via shear stress during bioprinting, Biofabrication 12 (2020) 045005) a gel paste formulation containing oxidized alginate and gelatin in combination to shear stresses is used to provide guidance to cell orientation and differentiation. The formulation of this prior art allows fast gelation which could be compatible high production capabilities. Nevertheless, the gel formulation does not contain any structuring agent and there is no active stretching applied to the fibers during production (the shear stress is limited to the shear at the exit of the ejection needle). Consequently, the fibers produced with this method do not have a fibrillar porous microstructure, as a consequence cell differentiation is poor and does not allow to realistically replicate the structure of muscle bundles.

In Kessel et al (3D Bioprinting of Macroporous Materials Based on Entangled Hydrogel Microstrands, Adv. Sci. 2020, 7, 2001419) a method to prepare anisotropic hydrogel fibers with microfibrillar structure is reported. There are two main two main limitations: (i) it requires to pre-crosslinked hydrogels containing cells and does not allow to start directly with a gel paste. The pre-crosslinking of the hydrogels is an additional time-consuming step that reduces cell viability and increase production time. Additionally, (ii) the pre-crosslinked hydrogels need to be extruded through small sieve (40-100um hole size). This will lead to clogging and reduction of cell viability during extrusion due to high shear stresses. Moreover, the chemical modifications applied to the biopolymer to allow the photocrosslinking are not cost-efficient for cultivated meat production yet.

In Zhang et al (Creating polymer hydrogel microfibres with internal alignment via electrical and mechanical stretching, Biomaterials, Volume 35, Issue 10, March 2014, Pages 3243-3251), authors report a strategy for the generation of hydrogel microfibres with internal alignment induced by a combination of electrical and mechanical stretching. Cells can be loaded directly in the hydrogel formulation cells (i.e., a seeding step is not required). Nevertheless, the process requires the use of high voltage (3000-5000V). The use of high voltage in processing/manufacturing facilities would pose risk for electric arcs and poses specific requirements in terms of electric insulation of machines. In addition to that, the reported process is relatively slow.

Also the use of bicarbonates in combination with cross-linkable polysaccharides and gelatine-based materials is possible. However, its use and function have been limited as gas-generating agent to produce porosity because of the $CO_2$ production. WO2006101453A1 discloses a method and composition to produce fibrous scaffolds by using interfacial polyelectrolyte complexation with the help of a Si-containing crosslinker. Scaffolding by using interfacial polyelectrolyte complexation is hardly scalable because it requires arranging multiple polyanion and polycation droplets and fiber formation is happening only at the interface between these droplets. This is hardly scalable for the production of cultivated meat. Additionally, the disclosed approach relies on electrostatic complexation.

No part of this discussion of the prior art is to be construed as an admittance of prior art.

SUMMARY OF THE INVENTION

Indeed, such anisotropic structure characteristic of animal muscle tissue made of muscle bundles (e.g., group of aligned muscle myofibrils) can be achieved only if the stem cells are guided in the differentiation process with the help of a suitable microenvironment, as well as topological and mechanical cues supporting myogenesis. In this invention, we disclose a method and compositions for the fabrication fibrillar hydrogels allowing the production of fibrous muscle bundles with characteristics like those of meat.

The disclosed invention comprises a method and compositions for the in-vitro production of muscle tissue for cultured meat production allowing the scalable production of muscle bundles of high quality and in large quantities. More specifically, the invention discloses (i) compositions for a gel paste which is turned within minutes into highly anisotropic fibrous gel embedding cells, (ii) a method for the fabrication of fibrous cell-laden gels, and (iii) compositions of the crosslinking baths used in the process. The fibrillar microstructure of the gel supports cell differentiation into muscle bundle with fibrillar and highly anisotropic texture. The major features are described below:

(i) The gel paste composition in combination with the method of production allows the fabrication of cell-laden composite gels characterized by unique anisotropic fibrillar structure at the microscale.

(ii) The production process to develop such fibrillar structure is fast and scalable and therefore compatible with the high production rate required for cultivated meat production.

(iii) The fibrillar structure of the composite gel promotes a superior differentiation of muscle progenitor stem cells into muscle tissue with the features typical of meat.

Advantages resulting from the major features of the invention comprise the following aspects, alone or in combination:

(1) Gel paste is composed of edible components.
(2) Differentiated cells assemble in muscle bundles with realistic size. Muscle bundles have a tunable diameter within 0.01 mm and 2 mm and a length tunable in size above 1 mm.
(3) Size of the muscle bundle can be tuned by playing on different process parameters. This allows the production of muscle fibers with a texture that mimics that of meat of different species.
(4) High cell density can be achieved, comparable to animal muscle tissue. Cell density up to hundreds of million cells per $cm^3$ can be achieved
(5) Cell seeding is efficient (with virtually 100% seeding efficiency) and homogeneous throughout the whole scaffold volume
(6) Gel paste composition and process parameters can be tuned to promote cell spreading and differentiation by fulfilling biomechanical requirements of any specific cell type.
(7) Fibrous gels embedding cells withstand culturing conditions for several weeks.
(8) Gel paste is converted into a mechanically stable gel within hundreds of seconds with cells being trapped and protected inside, making gel fibers easy to handle and compatible with a high production rate. Muscle bundles production can be completely automated, and production can happen directly inside the differentiation bioreactor.
(9) As a consequence of the fibrous texture of the gel entrapping cells, the resulting muscle bundles develop superior aligned fibrillar structure even without active tension during culture.
(10) High increase of protein content is observed upon differentiation as a consequence of the highly anisotropic gel microstructure and cell differentiation
(11) Mild processing conditions

Secondary/optional features consist of:

(12) Optionally, muscle bundles can be physically clamped during production in order to straightforwardly apply external stimuli (e.g. mechanical stretching and/or electrical field) to further push myofibrillar/myoglobin protein production.
(13) Optionally, non-proteinaceous components used as processing aids in the preparation of the gel paste (e.g., polysaccharide components) can be degraded/dissolved at the end of the differentiation process to maximize of the protein content and minimize the ingredient list.
(14) Optionally, bioactive molecule can be immobilized in the gel to support differentiation and spreading.
(15) Optionally, chromophores, flavour and aroma molecules can be added to the gel paste to improve overall sensory attributed of the produced meat.
(16) Optionally, fibroblasts can be seed on and/or in the muscle bundle to deposit connective tissue and further tune the texture of the final meat product
(17) Optionally, the crosslinking bath used for the gelation of the gel paste can contain iron ions to increase the iron content of the final meat product.
(18) Optionally, post-cultivation, muscle bundles can be glued together by using microbial transglutaminase. Furthermore, plant proteins and animal proteins can be added to tailor texture and protein content/source.
(19) Optionally, the produced muscle bundle, can be combined with plant, animal and cultivated fat to produce meat-based foodstuffs.
(20) Optionally, micro/nano fibres can be added in the gel paste formulation to increase the toughen the hydrogel and modulate texture.

So the present invention discloses methods and compositions for the fabrication of a cell-laden composite gel which can be formed on-demand with fibrillar structure. The invention is composed of different elements: a composition for a gel paste, and a method to produce gel fibers from the gel paste and the use of it to produce cultivated meat products.

More specifically speaking, according to a first aspect of the present invention, it relates to a paste for the fabrication of cultivated meat, comprising or composed of the following components:

(A) at least one polysaccharide crosslinkable to form a gel by the action of divalent or polyvalent cations, light-induced addition and/or condensation reaction, or a combination thereof, in a concentration in the paste in the range of 0.01-200 g per L of component (D);
(B) at least one protein assembling with the polysaccharide of component (A) via supramolecular or covalent interaction or a combination thereof, preferably selected from the group consisting of gelatin, collagen, fibrinogen, fibrin, fibronectin, fibroin, elastin, laminin, cardosin A, albumin, globulin, casein from milk as well as albumins, globulins, prolamins, glutenins, isolated from plant proteins, each in natural, synthetic, or recombinant form, as well as combinations thereof or consisting of such a naturally occurring full sequence or a fraction thereof, in a concentration in the paste in the range of 0.001-500 g per L of component (D);

(C) cells selected from mammalian cells, fish cells, crustaceous cells, or a combination thereof, preferably selected from embryonic stem cells, adult stem cells, induced stem cells, fibroblasts, fibrocytes or a combination thereof, in a concentration in the paste in the range of 0-300 billion cells per L of component (D);

(D) water or a water-based culturing medium;

(E) additives different from (A)-(D), selected from the group consisting of crosslinking kinetic modifier in a concentration in the paste in the range of 0-500 mM, flow modifier in a concentration in the paste in the range of 0-200 g per L of component (D), or a combination thereof.

According to a preferred embodiment of such a paste, the at least one polysaccharide of component (A) is naturally crosslinkable and/or chemically modified to promote crosslinking, wherein preferably the chemical modifications include acrylation, methacrylation, epoxidation, allylation, or a combination thereof.

According to a further preferred embodiment of such a paste, the at least one polysaccharide of component (A) is selected from the group consisting of alginates, pectins, carrageenans, chondroitin sulfate, dermatan sulfate, heparin, heparin sulfate, as well as derivatives thereof and combinations thereof, preferably the polysaccharide component of component (A) is at least one alginate or alginate derivative.

Preferably, the polysaccharide(s) of component (A) is present in a concentration in the paste in the range of 0.1-100 g per L of component (D).

According to yet another preferred embodiment the at least one protein of component (B) takes the form of a native protein, denatured protein, protein hydrolysate, or a combination thereof.

Preferably the at least one protein of component (B) is bioactive with the ability to modulate a function and/or characteristic of the cells of component (C), in particular to promote cell attachment by providing integrin binding motifs.

The at least one protein of component (B) can be preferably selected from the group consisting of gelatin, collagen, fibrinogen, fibrin, fibronectin, fibroin, elastin, laminin, basic albumins from plant, preferably napin, wherein preferably the protein component (B) is gelatine, more preferably gelatine, type A.

Preferably, the at least one protein of component (B) is chemically modified to promote crosslinking, wherein preferably the chemical modifications include acrylation, methacrylation, epoxidation, allylation, or a combination thereof.

According to a preferred embodiment, the at least one protein of component (B) is crosslinked, preferably by transglutaminase, peroxidase, laccase, tyrosinase, lysyl oxidase, glutaraldehyde, genipin, citric acid, photopolymerization or a combination thereof, preferably by transglutaminase is used, and wherein if transglutaminase is used as crosslinker, it is comprised in an amount of 0-50 U per g of protein, more preferably 0.1-10 U per g of protein.

Typically, the protein(s) of component (B) is present in a concentration in the paste in the range of 0.1-250 g per L of component (D).

According to yet another preferred embodiment, the cells of component (C) are present in a concentration in the paste in the range of 5-100 billion cells per L of component (D). The additives of component (E) preferably include at least one crosslinking kinetic modifier to slow down the gelation kinetics of the crosslinkable polysaccharide by sequestering divalent or polyvalent cations.

Preferably the additives of component (E) include at least one food-compatible compound selected from the group consisting of disodium phosphate, dipotassium phosphate, magnesium phosphate, ethylenediaminetetraacetic acid, ethylenediaminetetraacetic salts, or a combination thereof.

Typically the additives of component (E) are present in a concentration 1-100 mM.

The additives of component (E) May include at least one flow modifier to impart a shear thinning behaviour to the gel past so that the higher viscosity in resting condition mitigate cell sedimentation, whereas the lower viscosity during ejection allows processability, wherein preferably said flow modifiers are edible fillers in the form of micro/nanofibers, preferably insoluble in culturing conditions, and are preferably composed of protein and/or polysaccharide different from the other components of the paste, wherein such edible filler is preferably present at a concentration of 0-200g per L of paste, and wherein further preferably edible polymers as flow modifier can be selected among gums, including gellan gums, guar gums, xanthan gums), PEGs, or combinations thereof, and wherein preferably edible polymers are present at a concentration of 0-50g per L of gel paste.

Preferably, such a paste is further supplemented with a micro-structuring agent, for unfolding of protein structure, preferably selected from the group consisting of bicarbonate, including sodium bicarbonate, potassium bicarbonate, ammonium bicarbonate, magnesium bicarbonate, or a combination thereof. Preferably the micro-structuring agent is added to the paste in an amount to lead to at a concentration ranging from 0.1-300 g per L of paste, preferably, of 1-200 g per L of paste. The micro-structuring agent is preferably added in powder form. The micro-structuring agent and the paste may also take the form of a kit-of parts, which is joined right before carrying out the method for making the microfibril structure as detailed below.

According to a further aspect of the present invention, it relates to a method for the manufacturing of a microfibril structure for the fabrication of cultivated meat, wherein

(a) a paste, preferably as defined above, is supplemented with a food compatible micro-structuring agent for unfolding of protein structure
(b) the mixture is extruded through an ejection nozzle with an ejection speed under the generation of a speed gradient downstream of said nozzle with a pulling speed larger than the ejection speed to form a drawn paste, and
(c) the drawn paste is immersed in a hardening bath. Preferably such a hardening bath comprises divalent or polyvalent cations or is an acidic bath (e.g. in case a cross-linking aid such as calcium carbonate or other carbonates of divalent or polyvalent ions is present in the gel, the hardening bath can be an acidic bath, see also Example 7.c. reported below). According to a first preferred embodiment, the micro-structuring agent used in step (a) is selected from the group consisting of bicarbonate, including sodium bicarbonate, potassium bicarbonate, ammonium bicarbonate, magnesium bicarbonate, or a combination thereof, and/or wherein the micro-structuring agent is added to the paste in an amount to lead to at a concentration ranging from 0.1-300 g per L of paste, preferably, of 1-200 g per L of paste, and/or wherein the micro-structuring agent is added in powder form.

According to yet another preferred embodiment of the method, the extrusion in step (b) takes place with a drawing factor, defined as the ratio of the ejection speed to the pulling speed, of at least 1.1, preferably at least 1.5.

Preferably the fiber for the drawing is blocked on a clamping platform allowing to apply the drawing factor by moving at least one of the ejection nozzle or the clamping platform at a pulling speed, wherein the movement of the camping platform can be manual or automated and wherein the movement of the camping platform can be linear or rotative, or a combination thereof.

Preferably, the hardening bath of step (c) comprises $Ca^{2+}$, $Mg^{2+}$, $Fe^{2+}$ and $Fe^{3+}$ or a combination thereof in water, preferably at a concentration in the range of 1-500 mM, more preferably between 20-100 mM.

The hardening bath of step (c) can be buffered at a pH ranging from 6 to 8, preferably by using HEPES buffer, wherein further preferably the HEPES buffer is used in a concentration comprised between 1-100mM.

Preferably, in step (b) the paste is extruded from a nozzle preferably with a circular geometry at the ejection point.

Further preferably, in step (b) the paste is extruded from a nozzle with a diameter in the range of between 10 to 5000 μm, more preferably 100-1000 μm.

In step (b) the pulling rate, measured relative to the nozzle (the relative speed is relevant, the nozzle can be stationary or the pulling plate can be stationary or both can be moving relative to each other), is preferably in the range of 0.01-100 m/min, more preferably between 0.1-10 m/min.

According to yet another preferred embodiment, either the hardening bath comprises a protein cross-linking agent or subsequent to step (c) the fibres are immersed in a protein cross-linking bath with such a cross-linking agent.

Preferably such a cross-linking agent is selected from the group consisting of transglutaminase, peroxidase, laccase, tyrosinase, lysyl oxidase, glutaraldehyde, genipin, citric acid, or a combination thereof, preferably transglutaminase, and wherein if transglutaminase is used, it is comprised in an amount of 1-2000 U per mL of crosslinking bath.

Further preferably cross-linking is performed at a temperature in the range of 20-45°C, preferably for a time span in the range of 10-120 min, more preferably 30-90 min.

Muscle bundles can be prepared by cultivation of cell-laden microfibrillated hydrogel fibers in an environment promoting myogenesis.

Preferably, the resulting fibres, preferably having an individual diameter in the range of 0.1-50 μm, preferably aligned in the same axis ± 40° with respect of the fiber axis, are converted into muscle tissue by culturing them in differentiation media designed for the used cell type, wherein if the fibres have been produced from a paste without cells, before culturing the fibres are seeded with cells. Preferably the fibers, e.g. in the form of bundles, are cross-linked together to obtain a solid edible structure, wherein cross-linking of the fibres can be effected by one or several cross-linking agents selected from the group consisting of transglutaminase, peroxidase, laccase, tyrosinase, lysyl oxidase, glutaraldehyde, genipin, citric acid, or a combination thereof, preferably transglutaminase

Normally, the resulting muscle tissue or assembly of is mixed with further constituent to form a consumer cultured meat product.

According to yet another aspect the invention relates to a cultured meat product based on a paste as defined above, and/or obtained using a method as defined above.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1 shows the synergic interplay between gel paste composition and process to generate highly anisotropic hydrogel consisting of multiple fibrils stretched along the fiber axis;

Fig. 2 shows how supramolecular complexes are formed due to the nature of the gel components leading to the formation of fibrils within the hydrogel thanks to the action of the shear induced by the gradient of speed and the increased degree of interaction promoted by the MSA;

Fig. 3 shows in a) cells encapsulated in the microfibrils of the anisotropic hydrogel and the resulting myofibrillar structure of the muscle bundle because of cell spreading and differentiation within microfibrils, and in b) a schematic representation of the method used to produce anisotropic gels with a fibrillar structure from a gel paste: (A) Gradient of gelation and active pulling to generate microfibrils; (B) gel fiber with microfibrillar structure consolidated by gel paste gelation;

Fig. 4 shows a fiber drawing by pulling the gel paste in space while crosslinking. Pulling applied by using a planer (A) or a rotative (B) clamping platform;

Fig. 5 in the upper row shows an illustration of the bioactivity factor (BF) and in the lower row of the microfibrillar factor (MF);

Fig. 6 shows a fluorescent image of differentiated muscle cells (Bovine adult stem cells) after staining with Calcein AM in E.1. Alive cells are reported In green (FTIC channel). Magnification = 4x; the scale bar is equal to 430 $\mu$m. The imaged region was randomly selected for every sample;

Fig. 7 shows Gene expression of MYH7 normalised by housekeeping genes (GAPH, HPRT1) of cultivated muscle tissue from E1 formulation and of 2D differentiated cell culture. Data are reported as folds of increase in comparison to an undifferentiated cell reference;

Fig. 8 shows a fluorescent image of differentiated muscle cells (Bovine adult stem cells) after staining with Calcein AM in E.2. Alive cells are reported in green (FTIC channel). Magnification = 4x; the scale bar is equal to 430 $\mu$m. The imaged region was randomly selected for every sample;

Fig. 9 shows a fluorescent image of differentiated muscle cells (Bovine adult stem cells) after staining with Calcein AM in D3. Alive cells are reported In green (FTIC channel). Magnification = 4x; the scale bar is equal to 430 $\mu$m. The imaged region was randomly selected for every sample;

Fig. 10 shows a fluorescent image of differentiated muscle cells (Bovine adult stem cells) after staining with Calcein AM in E.4. Alive cells are reported In green (FTIC channel). Magnification = 4x; the scale bar is equal to 430 $\mu$m. The imaged region was randomly selected for every sample. Reported image was collected after 7 days of differentiation instead of 4 for reasons of force majeure. Nevertheless, estimation of MF and BF was always based on assessment after 4 days of differentiation;

Fig. 11 shows a fluorescent image of differentiated muscle cells (Bovine adult stem cells) after staining with Calcein AM in E.6. Alive cells are reported In green (FTIC channel). Magnification = 4x; the scale bar is equal to 430 $\mu$m. The imaged region was randomly selected for every sample;

Fig. 13 shows a fluorescent image of differentiated muscle cells (Bovine adult stem cells) after staining with Calcein AM in E.7. Alive cells are reported In green (FTIC channel). Magnification = 4x; the scale bar is equal to 430 $\mu$m. The imaged region was randomly selected for every sample;

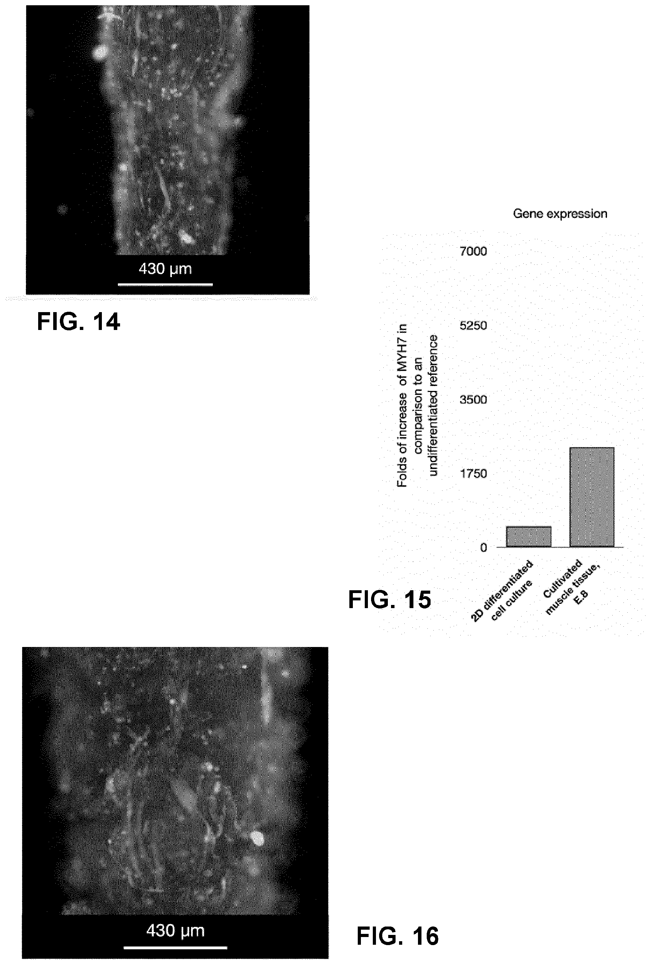

Fig. 14 shows a fluorescent image of differentiated muscle cells (Bovine adult stem cells) after staining with Calcein AM in E.8. Alive cells are reported In green (FTIC channel). Magnification = 4x; the scale bar is equal to 430 $\mu$m. The imaged region was randomly selected for every sample;

Fig. 15 shows gene expression of MYH7 normalised by housekeeping genes (GAPH, HPRT1) of cultivated muscle tissue from E1 formulation and of 2D differentiated cell culture. Data are reported as folds of increase in comparison to an undifferentiated cell reference;

Fig. 16 shows a fluorescent image of differentiated muscle cells (Bovine adult stem cells) after staining with Calcein AM in E.9. Alive cells are reported In green (FTIC channel). Magnification = 4x; the scale bar is equal to 430 $\mu$m. The imaged region was randomly selected for every sample;

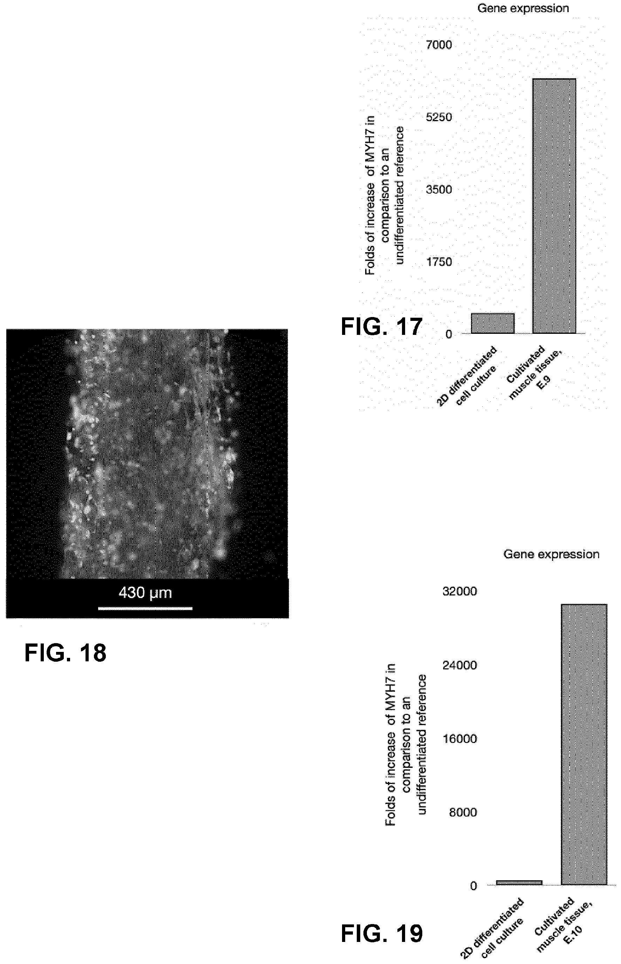

Fig. 17 shows gene expression of MYH7 normalised by housekeeping genes (GAPH, HPRT1) of cultivated muscle tissue from E9 formulation and of 2D differentiated cell culture. Data are reported as folds of increase in comparison to an undifferentiated cell reference;

Fig. 18 shows a fluorescent image of differentiated muscle cells (Bovine adult stem cells) after staining with Calcein AM in E. 10. Alive cells are reported In green (FTIC channel). Magnification = 4x; the scale bar is equal to 430 $\mu$m. The imaged region was randomly selected for every sample;

Fig. 19 shows gene expression of MYH7 normalised by housekeeping genes (GAPH, HPRT1) of cultivated muscle tissue from E10 formulation and of 2D differentiated cell culture. Data are reported as folds of increase in comparison to an undifferentiated cell reference;

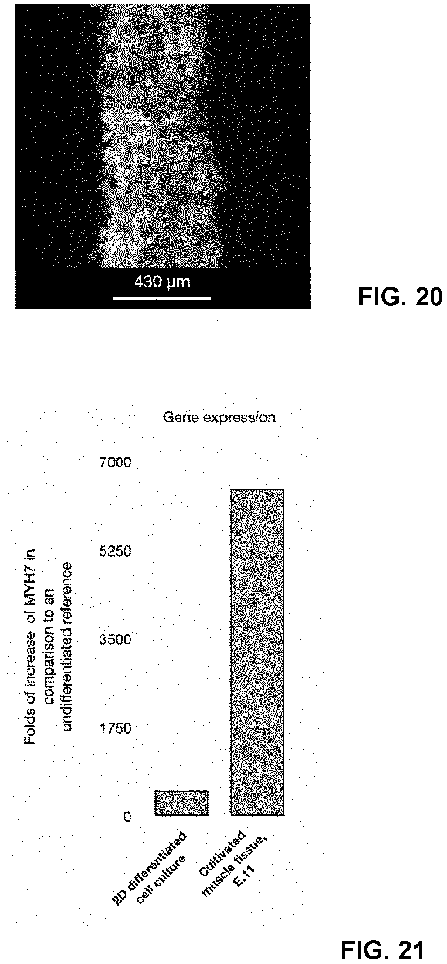

Fig. 20 shows a fluorescent image of differentiated muscle cells (Bovine adult stem cells) after staining with Calcein AM in E.11. Alive cells are reported In green (FTIC channel). Magnification = 4x; the scale bar is equal to 430 $\mu$m. The imaged region was randomly selected for every sample;

Fig. 21    shows gene expression of MYH7 normalised by housekeeping genes (GAPH, HPRT1) of cultivated muscle tissue from E11 formulation and of 2D differentiated cell culture. Data are reported as folds of increase in comparison to an undifferentiated cell reference;

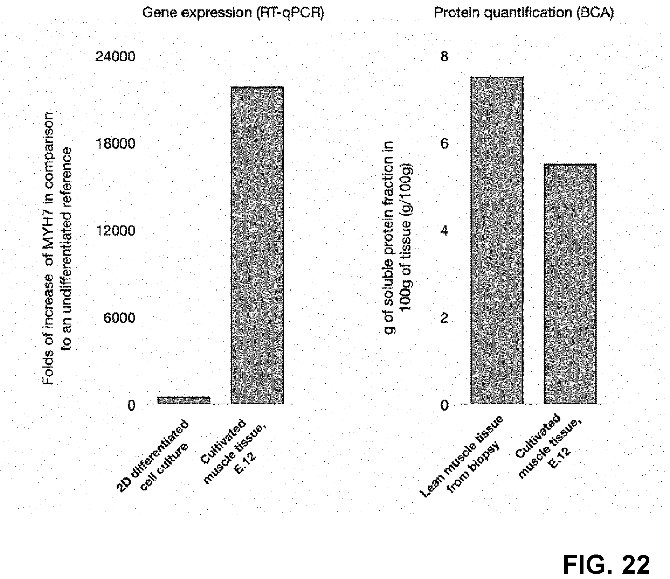

Fig. 22    shows gene expression of MYH7 normalised by housekeeping genes (GAPH, HPRT1) of cultivated muscle tissue from E1 formulation and of 2D differentiated cell culture. Data are reported as folds of increase in comparison to an undifferentiated cell reference. BCA assay for soluble protein fraction of cultivated muscle tissue from E.12 formulation in comparison to lean muscle tissue from biopsy.

## DESCRIPTION OF PREFERRED EMBODIMENTS

In the following an explanation of formation mechanism of micro-fibrillar structure at the hydrogel level shall be given. However it is to be noted that the corresponding description is not to be given a limiting interpretation, the causes and effects underlying the occurring phenomenon of microfibrillar structure formation are not limited to those listed below. Other causes present in the current experimental set-up but not isolated as causing factor may be responsible of additional effects responsible of the microfibrillar structure development or they might have a synergic/enhancing effect.

Any variation falling within the same processing principle (gel paste ejection and active pulling in a crosslinking bath) yet required due to specific needs, scale-up, scale-out, and automation are also considered within the scope of the present invention.

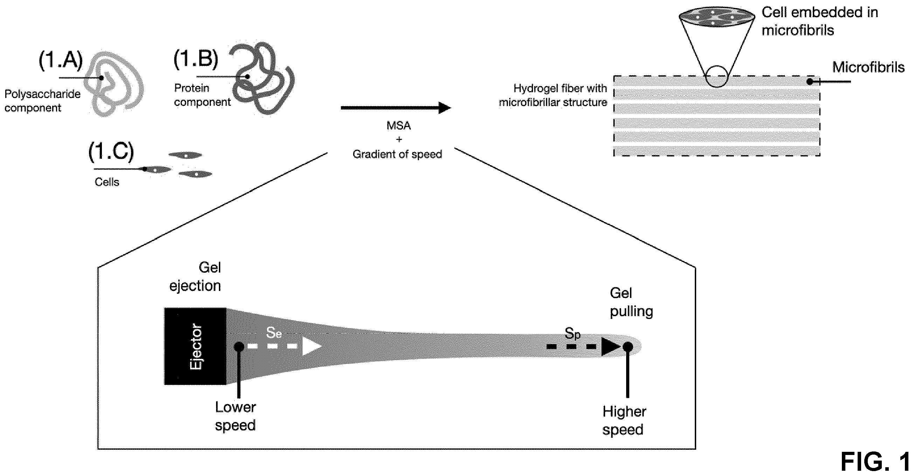

One core element of the invention is the scalable and food-compatible principle to generate fibrous anisotropy in hydrogel embedding cells. The features of the so generated hydrogel are responsible of the formation of muscle bundles (upon myogenesis) with morphological and nutritional features of muscle isolated from living animals. The principle of generation of the fibrillar structure at the hydrogel level is schematically illustrated in **Figure 1.**

The fibrillar structure is generated by the supramolecular interactions between the polysaccharide and protein components.

The nature of the interaction depends on the specific protein and its isoelectric point. These supramolecular interactions are of attractive nature and due to electrostatic interactions, H-bonding and hydrophobic interactions occurring between the amino acid residues of the protein and the sugar units of the polysaccharide. In the generation of the microfibrillar structure, the actions of the micro structuring agent (MSA) and the gradient of speed are responsible (but not limited) to the following aspects:

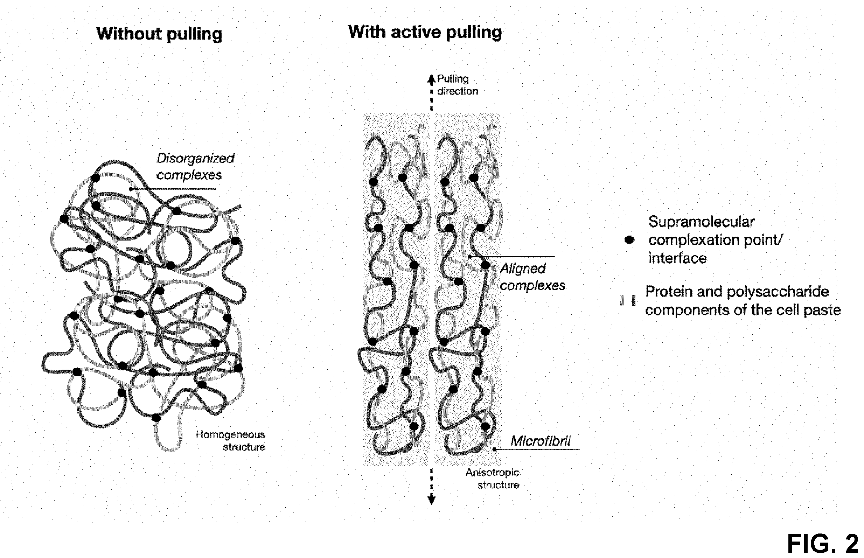

- Enhanced complexation between polysaccharide and protein components: The bicarbonate ions and the generated $CO_2$ in combination with the shear (due to the gradient of speed) are responsible of the unfolding of the protein structure. This exposes a large surface area which can then interact via supramolecular interactions with the polysaccharide. These interactions lead to the formation of microfibrils via the action of the pulling thanks to the occurrence of interfacial complexation (**Figure 2**)

- Microporosity development: The generation of $CO_2$ during the myofibrillar structure development results in the formation of a porous network that promotes cell spreading and differentiation.

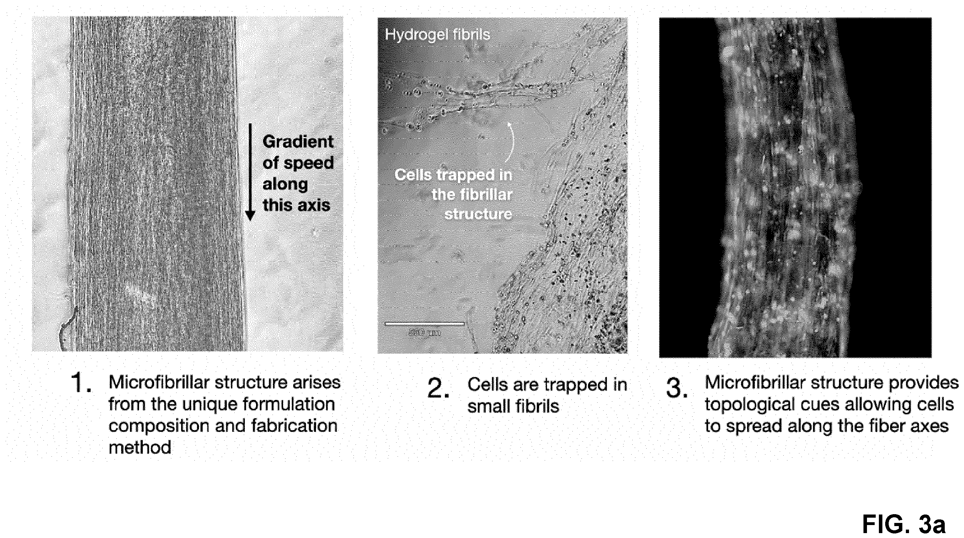

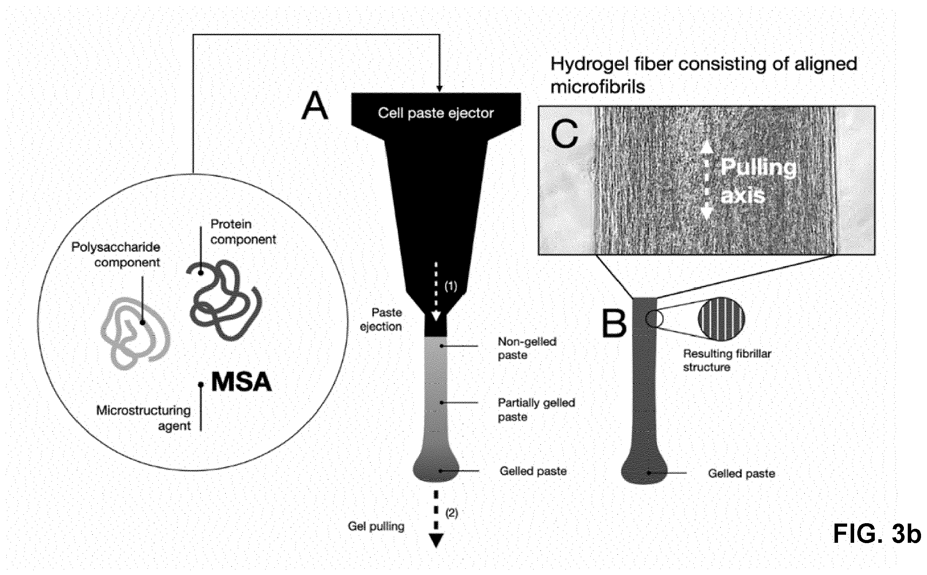

When cells are added to the gel formulations, cells result embedded within the small fibrils. This embedment forces cell spreading and differentiation to occur along the fibril axis. As a consequence, the gel will turn into a highly anisotropic cellular construct equivalent to the anatomy of a muscle bundle, **Figure 3.**

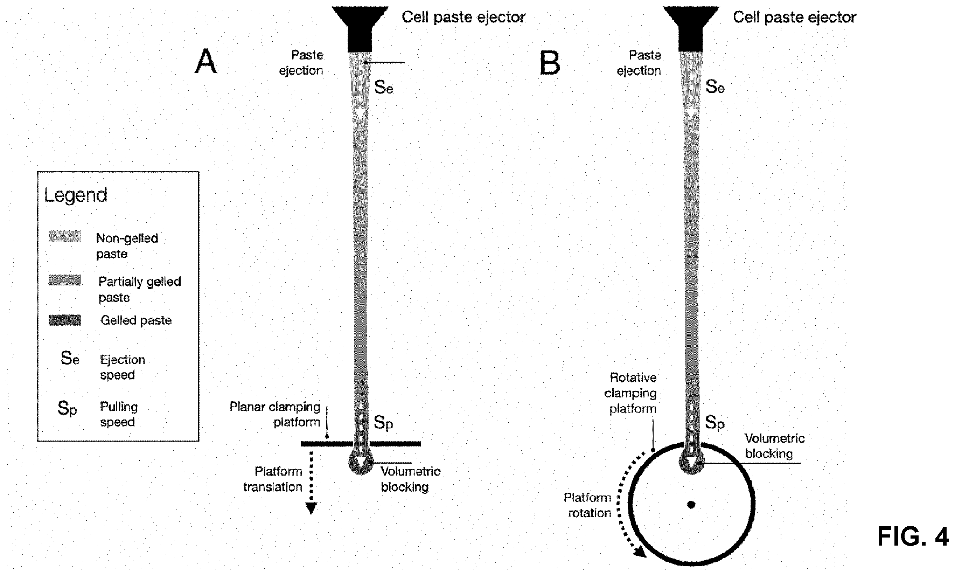

The drawing factor can be induced by pulling the fiber during crosslinking. This results in an acceleration of the gel paste and the crosslinking freezes the generated fibrillar structure. An example is reported in **Figure 4.**

For this methodology, the (gel) paste is first blocked to the clamping platform. Once the blocking is generated, the gel paste starts being pulled (in a crosslinking bath) in order to induce a gradient of speed. The gradient of speed is controlled by tuning the speed of ejection the speed of the partially/fully gelled paste at the level of the clamping platform. The pulling can be induced by a translational (Figure 4A) or rotative (Figure 4B) movement of the clamping platform. Multiple fibers can be produced in parallel by designing clamping platforms with multiple holes. In specific embodiments a top clamping platform can be also used in order to block the fiber(s) between two points. This set up can be used when it is necessary to apply stimuli to promote differentiation (e.g., active tensioning, cyclic stretching, electrical stimulations, etc.). Note that for gradient of speed by pulling in space the MSA plays another beneficial role of slowing down the polysaccharide gelation, allowing bigger speed gradients before gelation occurs. Indeed, the bicarbonate ions can sequester cations from the crosslinking bath. This is translated in a slower gelation kinetics since new cations must diffuse in to crosslink the polysaccharide. By slowing down the kinetics of crosslinking it is possible to impart a better alignment by applying a larger speed gradient.

**Experimental evidence**

Overview

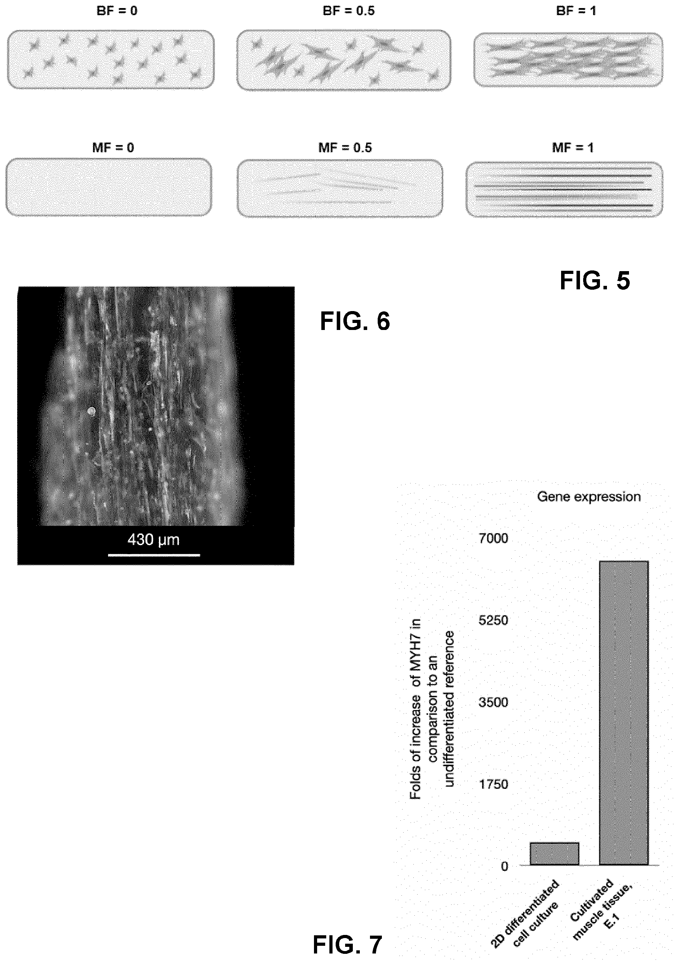

In the following, experimental details for eleven different formulations are reported. Details regarding the composition of each single formulation, processing conditions, results and discussion are reported in the "Example" sections. The quality of a formulation can be assessed on the basis of different factors. For this purpose, we defined two factors (BF and MF) which were the used to compute an overall score (OS) as detailed below (see also **Figure 5**):
BF, Bioactivity Factor (it considers the cell behavior, which is related to the nutritional quality of the produced bundles)

- BF=1, extensive cell spreading observed + superior cell alignment + differentiation better than 2D control. Differentiation is considered superior with respect to the control if the MHY7 expression is higher than 2-fold increase in comparison to the control
- BF=0.5, partial cell spreading observed + poor cell alignment
- BF=0, very poor cell spreading observed and/or poor cell viability MF, Microfibrillar Factor (it takes into account the structural aspects of the hydrogel, which are related to the texture of the produced muscle bundles)
- MF=1, complete microfibrillar internal structure with superior alignment
- MF=0.5, partial microfibrillar internal structure with no complete alignment
- MF=0, homogeneous hydrogel fiber without visible internal fibrillar structure OS, Overall score (it takes into account both the nutritional and sensorial aspect of the cultivated muscle bundles)

$$OS = (MF + 1.5BF)/2.5$$

BF is weighted 50% more than MF because differentiation performance will determine the nutritional attributes of cultivated meat which are the most important aspect of the final product as intended for human nutrition.
**Table 1.** Details: All % are referring to weight % with respect to the total weight of water. (B) are the protein components, (A) is the crosslinkable polysaccharide, MSA is the microstructuring agent, EX (3.7) is referring to fibers which were enzymatically crosslinked in transglutaminase bath, IX ((1.3).7) is referring to fibers which were produced from formulations containing transglutaminase. Not/u is reported when a component was not used in the formulation. Saline crosslinking bath is used in all cases during fiber drawing.

| | Polysaccha ride component(A) | Optional compon ent | MS A | Speed gradie nt Sp/Se | Protein Crosslink ing | Example Formulat ion | Microfibri liar factor (MF) | Bioactiv ity factor (BF) | |
|---|---|---|---|---|---|---|---|---|---|
| **Protein compon ent (B) at 4%** | 2% | not/u | 10 % | 1.9 | EX | 9 | 0.75 | 0.75 | **0.75** |
| | 2% | not/u | 10 % | 1.9 | IX/EX | 1 | 1 | 0.85 | **0.91** |
| | 2% | hydro lys ate 0.5% | 10 % | 1.9 | IX/EX | 10 | 0.85 | 1 | **0.94** |
| | 2% | hydro lys ate 2% | 10 % | 1.9 | IX/EX | 11 | 0.85 | 0.75 | **0.79** |
| **Protein compon ent (B) at 8%** | 2% | not/u | - | 1.9 | IX/EX | 7 | 0 | 0 | 0 |
| | 2% | not/u | 10 % | n/a | IX/EX | 2 | 0.1 | 0.15 | **0.13** |
| | 2% | not/u | 10 % | 1.9 | IX/EX | 8 | 0.65 | 0.65 | **0.65** |
| | 2% | not/u | 10 % | 1.9 | EX | 3 | 0.9 | 0.75 | **0.81** |
| | 2% | Nanofibe rs 1% | 10 % | 1.9 | IX/EX | 5 | 0.6 | 0.6 | **0.6** |
| | 2% | Nanofibe rs 3% | 10 % | 1.9 | IX/EX | 6 | 0.6 | 0.55 | **0.57** |
| | 2% | - | 20 % | 1.9 | IX/EX | 4 | 0.95 | 0.8 | **0.86** |

The following general comments are to be considered:

- Fibers produced without active pulling, but simply ejecting the gel paste in a crosslinking solution (ionic crosslinking), resulted in an OS_0. This confirms that minimal drawing factor is a necessary element of the invention (E.2).
- Fibers produced without MSA did not show a fibrillar texture and cells could not spread. This confirms that MSA is an important element of the invention. OS=0
- Different content of protein components can be used. This indicates that based on the specific needs, the protein content of the scaffolding components can be tuned
- Formulations with low content of the protein component work better with enzymatic protein crosslinking agent in the formulation plus a further step of external protein crosslinking. (IX and EX)
- Formulations with high content of the protein component (E.8, E.3) work better without enzymatic crosslinker in the formulation (i.e., no IX). In this case, an enzymatic external protein crosslinking (EX) is sufficient to guarantee the stiffness modulation required for cell spreading.
- Increasing the content of MSA (E.3 vs E.4) is beneficial for both BF and MF.
- Nanofibers (prolamine-based) can be added in the formulation to the tailor texture and tune the hydrogel structure (e.g., E.5, E.6)
- Peptide/polypeptides known to boost muscle cell differentiation can be added in the formulation as a structural component of the hydrogel to further promote differentiation performances (E.10, E11).
- Formulations containing exclusively the crosslinkable polysaccharide (A) without any protein component (B) could not generate microfibrillar structure (Experiments done without cells, not reported here). This confirms that the protein component is a necessary element of the invention.
- Formulations containing exclusively the protein component (B) without any crosslinkable polysaccharide (A) cannot be used to produce hydrogel fibers (Experiments done without cells, not reported here). This confirms that crosslinkable polysaccharide is a necessary element of the invention.

Equipments:

Positive displacement pipette (Gibson), Glass syringe 2 ml (Darwin Microfluidics), needles 18G (RameArt), perforated metallic plate used as clamping platform. Reagent, chemicals and solutions: Gelatin porcine type A (Sigma Aldrich, G2500), Sodium Alginate (Sigma Aldrich, 180947), Microbial Transglutaminase concentrate (mTGase) - 2000 U/g (BDF Ingredients, ProBIND TXo 17.0), Sodium bicarbonate (Sigma Aldrich, S5761), Calcium Chloride (Sigma Aldrich, C1016), MilliQ water, HEPES 1M (Sigma Aldrich, H3537), glutenin/gliadin hydrolysate (hereafter just called hydrolysate), prolamine-based nanofibers (hereafter just called nanofibers).

Analysis performed:

Cell imaging, gene expression, and BCA protein assay have been conducted after 4 days of differentiation, unless otherwise specified.

- Qualitative imaging (fluorescent microscopy)
  Cells were stained by using Calcein-AM (C3100MP, Fisher Scientific) live staining and visualised under fluorescence microscope in FITC channel (LED) in uninverted setup at 4x magnification.
- Gene expression (qPCR) of MYH7
  Total RNA samples were isolated from adherent or pelleted cells using a commercial RNA-extraction kit from Zymo Research. The RNA should be of good quality (A260/230 > 1.7 and A260/280 between 1.8 - 2.2). Gene expression analysis was executed by using a qPCR thermal cycler (Azure, Cielo 6).
- BCA protein assay
  Tissues were homogenised at 15000 rpm for 1 minute in 2.5% SDS. The supernatant was isolated and analysed with BCA Protein Assay kit (71285-3, supplier). Quantification was based on calibration curves performed on Bovine Serum Albumin standard in 2.5% SDS.

Formulation overview:

Table 2: Gel paste formulation and processing parameters.

| Example Formulation n° | Liquid (D) | Main protein component 1 (1B) | Cross-linkable polysacch aride component (A) | Optional component | Micro-structuring agent (MSA) | Enzymati c IX Crosslin king agent | Cells (C) | Ejection speed (m/min) | Pulling speed (m/min) |
|---|---|---|---|---|---|---|---|---|---|
| 1 | Ultrapu re Water/ 1 ml | Gelatin A 40 mg | Sodiu m Alginat e 20 mg | - | Sodium Bicarbon ate,100 mg | mTGase, 50 mU | 18 millions | 0.72 | 1.4 |
| 2 | Ultrapu re Water/ 1 ml | Gelatin A 80 mg | Sodiu m Alginat e 20 mg | - | Sodium Bicarbon ate, 100 mg | mTGase, 100 mU | 18 millions | 0.72 | n/a |
| 3 | Ultrapu re Water/ 1 ml | Gelatin A 80 mg | Sodiu m Alginat e 20 mg | - | Sodium Bicarbon ate, 100 mg | - | 18 millions | 0.72 | 1.4 |
| 4 | Ultrapu re Water/ 1 ml | Gelatin A 80 mg | Sodiu m Alginat e 20 mg | - | Sodium Bicarbon ate, 200 mg | mTGase, 100 mU | 18 millions | 0.72 | 1.4 |
| 5 | Ultrapu re Water/ 1 ml | Gelatin A 80 mg | Sodiu m Alginat e 20 mg | Prolam ine-based nanofi bers 10 mg | Sodium Bicarbon ate, 100 mg | mTGase, 100 mU | 18 millions | 0.72 | 1.4 |
| 6 | Ultrapu re Water/ 1 ml | Gelatin A 80 mg | Sodiu m Alginat e 20 mg | Prolam ine-based nanofi bers 30 mg | Sodium Bicarbon ate, 100 mg | mTGase, 100 mU | 18 millions | 0.72 | 1.4 |
| 7 | Ultrapu re Water/ 1 ml | Gelatin A 80 mg | Sodiu m Alginat e 20 mg | - | - | mTGase, 100 mU | 18 millions | 0.72 | 1.4 |
| 8 | Ultrapu re Water/ 1 ml | Gelatin A 80 mg | Sodiu m Alginat e 20 mg | - | Sodium Bicarbon ate, 100 mg | mTGase, 100 mU | 18 millions | 0.72 | 1.4 |
| 9 | Ultrapu re Water/ 1 ml | Gelatin A 40 mg | Sodiu m Alginat e 20 mg | - | Sodium Bicarbon ate, 100 mg | - | 18 millions | 0.72 | 1.4 |
| 10 | Ultrapu re Water/ 1 ml | Gelatin A 40 mg | Sodiu m Alginat e 20 mg | Hydrol ysate 5 mg | Sodium Bicarbon ate, 100 mg | mTGase, 50 mU | 18 millions | 0.72 | 1.4 |
| 11 | Ultrapu re Water/ 1 ml | Gelatin A 40 mg | Sodiu m Alginat e 20 mg | Hydrol ysate 20 mg | Sodium Bicarbon ate, 100 mg | mTGase, 50 mU | 18 millions | 0.72 | 1.4 |
| 12 | Ultrapu re Water/ 1 ml | Gelatin A 80 mg | Sodiu m Alginat e 20 mg | - | Sodium Bicarbon ate, 100 mg | - | 18 millions | 0.72 | 1.4 |

EP 4 252 549 A1

12

Crosslinking baths:

In typical procedure, 1 ml of hydrogel is crosslinked in 20 ml of crosslinking bath.

This is valid for every Example of this patent where enzymatic EX crosslinking and Saline crosslinking are involved.

| Enzymatic EX crosslinking agent - concentration | Saline (ionic) crosslinking bath - concentration |
|---|---|
| mTGase -125 U/ml of bath | Calcium chloride - 100 mM, HEPES 20 mM |

## Example 1 (E.1)

PROCEDURES

All the following steps need to be executed under sterile conditions. All the non-single-use equipment needs to be autoclaved to let everything be sterile. Media and buffer solutions need to be pre-warmed-up for 1h at 37°C.

**Step 1:** Stock solution preparation and sterilisation

**Protein component 1, stock (B):**

Pour 20 ml of HEPES 5 mM in a 50 ml Falcon tube; heat it at 37°C in the water bath prior to Gelatin A addition for 30 minutes.

Weigh 1.6 g of Gelatin A on a weight scale.

Transfer it in 20 ml HEPES. Note: add the powder gradually to avoid clumps formation. Solubilize the solution at 60°C on the heating plate for 30 minutes; vortex the mixture frequently to improve the mixing.

Store the solution in the fridge for further usage.

Before use, the solution should be filtered with a sterile syringe filter (0.22 um). Note: before filtration, warm up the solutions at a temperature above 60°C and prewarm syringe and syringe filters at 37°C. This is necessary to avoid gelation during filtration. Experiencing strong resistance from the syringe piston during filtration is normal, continuing to provide a steady pressure on the piston. The filtration of a 20mL solution can take up to dozens of minutes.

Prior use, place them in the water bath for 1 h to let them reach 37°C. Mix from time to time to ensure homogeneous temperature distribution and avoid the formation of concentration gradients.

**Crosslinkable polysaccharide component, stock (A):**

Pour 20 ml of HEPES 5 mM in a 50 ml Falcon tube; heat it at 37°C in the water bath prior Alginate addition for 30 minutes.

Weight 0.8g of sodium alginate on a weight scale.

Transfer it in 20 ml HEPES. Note: add the powder gradually to avoid clumps formation. Solubilize the solution at 60°C on the heating plate for 30 minutes; vortex the mixture frequently to improve the mixing.

Store the solution in the fridge for further usage.

Before use, the solution should be filtered with a sterile syringe filter (0.22 um). Note: before filtration, warm up the solutions at a temperature above 60°C and prewarm syringe and syringe filters at 37°C. This is necessary to avoid gelation during filtration. Experiencing strong resistance from the syringe piston during filtration is normal, continuing to provide a steady pressure on the piston. The filtration of a 20mL solution can take up to dozens of minutes.

Prior use, place them in the water bath for 1 h to let them reach 37°C. Mix from time to time to ensure homogeneous temperature distribution and avoid the formation of concentration gradients.

**Enzymatic internal crosslinker stock (IX):**

Take an aliquot of mTGase from -20°C and let it warm to RT.

Weight 50 mg of mTGase concentrate and transfer them in a 2mL Eppendorf tube; this will result in a stock solution with 50 U/ml.

Pour 2 ml of HEPES 5 mM in the tube containing the mTGase;

Vortex for 10-30 sec

The IX should be prepared just before starting the preparation since the enzyme is not stable. Store the stock at RT prior to its use.

**Step 2:** Crosslinking baths preparation

**Saline crosslinking bath:**

Pour 1 L of milliQ in a plastic bottle.

Weigh 11.1 g of CaCl2 powder on the weight scale.

Transfer them into 1 L of milliQ water.

Add the required amount of HEPES 1 M to reach a final concentration of 20 mM

Shake the bottle to help mixing process.

Sterilise the hardening bath by using a new bottle with a filter on top.

**Enzymatic crosslinking bath (EX):**

Take an aliquot of mTGase from -20°C and let it warm to RT.

Weight 125 mg of mTGase concentrate and transfer them in a 50mL tube.

Pour 20mL of cell media in the tube containing the enzyme and mix well.

Sterile filter the solution with a 0.22um syringe filter.

**Step 3:** Micro-structuring agent (MSA) preparation and sterilisation

Weight an amount of sodium bicarbonate to reach 100mg for each mL of hydrogel volume and place in on a weighing boat.

Place it in the UV box for 1200 seconds.

Quickly transfer it to an Eppendorf tube.

**Step 4:** Cell detachment and preparation

The right number of cells is pelleted by centrifugation at 350 g at room temperature for 10 min;

Remove all the supernatant carefully without touching the cell pellet

Place the pellet in the water bath to keep it at 37°C while preparing materials' solutions. At this point, cells are ready to be encapsulated in the hydrogel formulation.

**Step 5:** Fiber ejection (for the ejection of 1 ml hydrogel)

Add 500 ul of (1.3) to the cells and slowly pipette up to avoid bubble formation, and slowly release all liquid trapped in the tip;

Add 500 ul of (1.2) to the tube containing (1.3); we will end up having a volume of 1 ml (1.3) 4% and (1.2) 2% with 18 million cells per mL).

Mix properly the hydrogel mixture and place the vial at 37°C for 5 minutes to equilibrate the gel paste temperature.

Add the enzymatic internal crosslinking (IX): 1 ul of IX stock solution to the 1 ml gel paste and mix; this corresponds to 1.25 mU of IX/mg of protein, resulting in 50 mU/ml of hydrogel.

Add the UV-sterilised sodium bicarbonate (100 mg) and mix quickly.

Load the hydrogel solution containing cells into a 2 ml glass syringe making use of a 18G sterile single use needles;

Place the 18G blunt-tip needle on the glass syringe.

Eject the fibers by using the perforated metallic plate (minimum hole diameter = 1.27 mm) in the saline crosslinking bath following the hereby mentioned precautions; note that the function of the plate is to allow the application of a pulling force at a specific pulling speed during ejection:

> Contact the plate-hole with the blunt tip and gently release gel paste to generate a hydrogel sphere/clump behind the plate. This will generate a steric blocking for the hydrogel, acting as a holding point for the fiber being ejected in the following steps. After having ejected the desired length, keep the hydrogel fiber under constant pulling for few seconds.

Cut the fiber gently with tweezers and transfer it in a saline hardening bath for 10 minutes (20mL in a petri dish) at RT.

**Step 6:** Enzymatic external crosslinking (EX)

Pour the EX bath in a Petri dish.
Transfer the cross-linked fibers from the saline bath to EX bath.
Place the petri dish in the incubator for 2h to let the enzymatic crosslinking to take place. Transfer the fibers in a 6 well-plate ULA.
add 5 ml/well of cell medium.
Place the well-plate in the incubator at 37°C on an orbital shaker (1-10 rpm).

**Step 7:** Maintenance

Media need to be refreshed according to proliferation and differentiation protocol.
In a typical procedure, the cell-laden microfibrillar hydrogels are converted into muscle bundles by inducing muscle cell differentiation. Due to experimental requirements related to cell culture, hydrogels are acclimated for a period ranging between 4 to 7 days prior inducing myogenesis. Myogenesis is induced by differentiation medium formulation.
In formulation E.1, the content of the protein (B) was observed to decrease from 8% to 4% in comparison to E.8. This was done with the intent of replicating the findings of E.4. In this intent, the ratio between biopolymers ((B)+(A)) and MSA was set to 1:1.7 (wt:wt) by keeping the concentration of biopolymers lower than previous formulations. As a consequence of the lower biopolymer content, the formulation showed lower viscosity and higher processability. Furthermore, the microfibrillar structure improved dramatically (MF=1) and the same happened for the overall differentiation performance.
Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). Formulation of Example 1 leads to an increase of differentiation performance of 13.2-folds in comparison to the reference (2D control), **Figure 7**.
This example demonstrates that formulation composition can be tuned in terms of biopolymers and MSA content to improve processability.

**Example 2 (E.2)**

Procedures as per E.1 with the following adjustments:

Step 1: for (1.3) stock solution, weight 3.2 g of Gelatin A on a weight scale.
Step 5:

> when adding 500 ul of (1.2) to the tube containing (1.3), we will end up having a volume of 1 ml ((1.3) 8% and (1.2) 2% with 18 million cells per mL).
> when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).
> no pulling is exerted here, so simply eject the fibers in the saline crosslinking bath.

Results: With the same composition of E.8 (see below), in E.2 we did not employ any active pulling during fiber production. Clearly because of the ejection through a nozzle, some degree of shear stresses will also be present in this case. Without the active pulling, the crosslinked formed an opaque fibrous hydrogel (suggesting that supramolecular interaction are still happening to some extent), but the fibrous texture of the hydrogel was chaotically oriented in all direction rather than in the form of a microfibrillar aligned structure as for fibers produced under pulling. At a cell level, qualitative imaging showed some degree of cell spreading. However, because of the poor cellular alignment, cells could not differentiate properly, and in differentiation medium we just observed a reduction of cell viability. This example confirms that pulling is a necessary element of the invention.

### Example 3 (E.3)

Procedures as per E.1 with the following adjustments:

Step 1:

for (1.3) stock solution, weight 3.2 g of Gelatin A on a weight scale;
no enzymatic IX stock needs to be prepared

Step 5:

when adding 500 ul of (1.2) to the tube containing (1.3), we will end up having a volume of 1 ml ((1.3) 8% and (1.2) 2% with 18 million cells per mL);
no addition of IX;

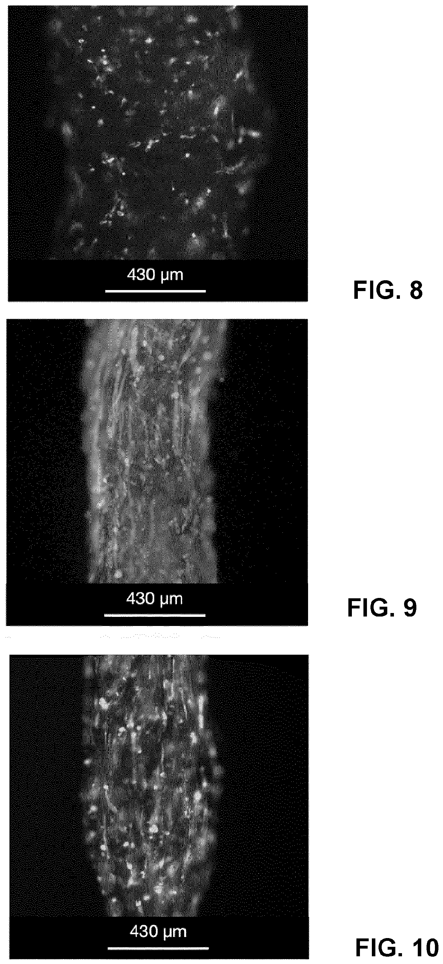

Results: With the same composition of E.8 (see below) but without any internal protein crosslinker, in E.3 we investigated the possibility to improve microfibrillar structure and processability. Without protein crosslinker the formulation showed a lower viscosity and a better stability overtime. A MF of 0.9 was identified from qualitative assessment. Additionally, as reported in **Figure 9**, cell viability was very high, cells could spread and differentiate in a highly anisotropic structure along the fiber axes. In conclusion, when a high protein component is used (B), internal crosslinker (IX) should not be employed or its concentration should be lowered.

### Example 4 (E.4)

Procedures as per E.1 with the following adjustments:

Step 1: for (1.3) stock solution, weight 3.2 g of Gelatin A on a weight scale.
Step 3: weight an amount of sodium bicarbonate to reach 200mg for each mL of hydrogel volume and place in on a weighing boat.
Step 5:

when adding 500 ul of (1.2) to the tube containing (1.3), we will end up having a volume of 1 ml ((1.3) 8% and (1.2) 2% with 18 million cells per mL).
when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).

Results: With a similar composition of E.8 (see below) but with higher MSA content (20% instead of 10%), in E.4 we tested out the influence of the structuring agent of the development of the microfibrillar structure. Overall, by increasing the ratio between biopolymers ((B)+(A)) and MSA from 1:1 (wt:wt) of E.8 to 1:2 of E.4, an improved microfibrillar structuration was observed (MF from 0.65 to 0.95) which in turns resulted in a improved differentiation performance. An exemplificative image is reported in **Figure 10.** This example suggests that when using internal crosslinking (IX) micro fibrillation can be improved by increasing the MSA content.

### Example 5 (E.5)

Procedures as per E.1 with the following adjustments:
Step 1: for (1.3) stock solution, weight 3.2 g of Gelatin A on a weight scale.
Intermediate step between 3 and 4:

Weigh nanofibers to reach 10 mg for each mL of hydrogel volume and place in on a weighing boat.
Quickly transfer it to an Eppendorf tube.
Step 5:

when adding 500 ul of (1.2) to the tube containing (1.3), we will end up having a volume of 1 ml ((1.3) 8% and (1.2) 2% with 18 million cells per mL).

after the first mixing of the hydrogel mixture with P1000, add the weighted optional component the hydrogel mixture and mix properly.

when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).

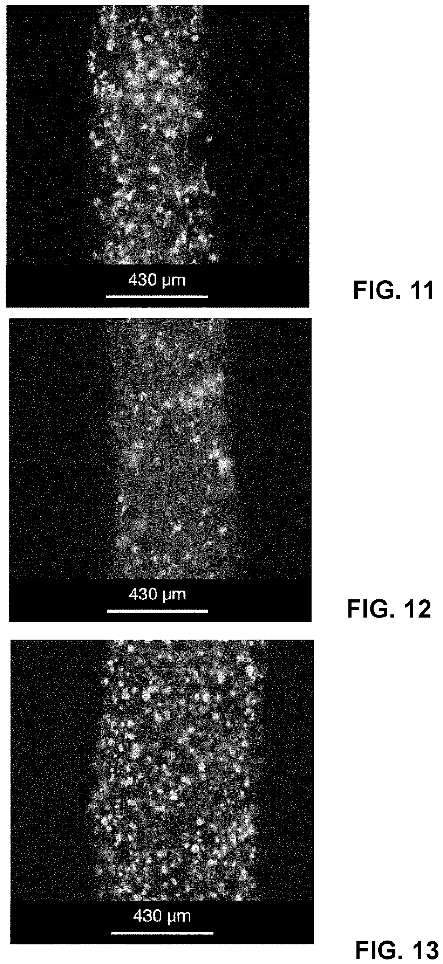

Results: E.5 formulation was designed with the same base composition of E.8 (see below) but with the addition of a nanofiber optional component. In E.5 we prove that protein nanofiber can be added to: (i) increase the overall protein content, (ii) enhance fibrous texture and rigidity. Nevertheless, the addition of nanofibers is worsening of the microfibrillar structure and of the differentiation performance (see **Figure 11**).

### Example 6 (E.6)

Procedures as per E.1 with the following adjustments:

Step 1: for (1.3) stock solution, weight 3.2 g of Gelatin A on a weight scale.

Intermediate step between 3 and 4:

Weight an amount of nanofibers to reach 30 mg for each mL of hydrogel volume and place in on a weighing boat. Quickly transfer it to an Eppendorf tube.

Step 5:

when adding 500 ul of (1.2) to the tube containing (1.3), we will end up having a volume of 1 ml ((1.3) 8% and (1.2) 2% with 18 million cells per mL).

after the first mixing of the hydrogel mixture with P1000, add the weighted optional component to the hydrogel mixture and mix properly.

when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).

Results: E.6 formulation was designed with even higher optional component content in comparison to E.5. The final score was very close to E.5 but with a worsening of the bioactivity factor. Although the overall differentiation performance was worse than E.8 (see below), this formulation can still be valuable for the preparation of specific foodstuff.

### Example 7 (E.7)

Procedures as per E.1 with the following adjustments:

Step 1: for (1.3) stock solution, weight 3.2 g of Gelatin A on a weight scale.

Step 3: absent.

Step 5:

when adding 500 ul of (1.2) to the tube containing (1.3), we will end up having a volume of 1 ml ((1.3) 8% and (1.2) 2% with 18 million cells per mL).

when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).

no MSA addition to hydrogel mixture.

Results: E.7. was designed based on E.8 (see below) but without any MSA. In the absence of MSA, only homogeneous bulky hydrogels could be formed; there was no sign of microfibrillar structure formation. Additionally, as reported in Figure 13, cells could not spread and therefore no differentiation was observed. This example proves that MSA is an important element for the development of the microfibrillar structure.

### Example 8 (E.8)

Procedures as per E.1 with the following adjustments:

Step 1: for (1.3) stock solution, weight 3.2 g of Gelatin A on a weight scale.
Step 5:

when adding 500 ul of (1.2) to the tube containing (1.3), we will end up having a volume of 1 ml ((1.3) 8% and (1.2) 2% with 18 million cells per mL).
when adding the enzymatic internal crosslinking (IX), 2 ul of IX stock solution need to be added to the 1 ml gel paste and mix; this corresponds to 1.25 mU of IX/mg of protein, resulting in 100 mU/ml of hydrogel (as mentioned in the formulation overview, paragraph 2).

Results: Fibers assessment showed a decent microfibrillar structure with a MF of about 0.65. Qualitative imaging of alive cells showed good cell spreading differentiation with a good degree of alignment along the fiber axis. Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). Formulation of Example 8 leads to an increase of differentiation performance of 4.8 in comparison to the reference (2D control). However, at this higher protein content, the gel paste is highly viscous and tends to crosslink rather quickly. For these reasons it is difficult to impart a good microfibrillar structure. For this purpose, we advise to not use an internally protein crosslinker (IX, mTGase). The same formulation of E.8 but without internal cross linking is reported in E.3.

### Example 9 (E.9)

Procedures as per E.1 with the following adjustments:

Step 1: no enzymatic IX stock needs to be prepared
Step 5: no addition of IX;
Results: With the same composition of E.1 but without any internal protein crosslinker (IX), in E.9 we investigated the role of the internal crosslinker for the formulation with low protein content ((B)). Qualitative assessment of the microfibrillar structure showed that the absence of the internal protein crosslinker has a negative effect on the microfibrillar structure of the hydrogel. Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). Formulation of Example 9 leads to an increase of differentiation performance of 12.5-folds in comparison to the reference (2D control). These folds of increase for E.9 were within the same range as for E.1. This suggests that in terms of differentiation performance not much is affected.

This example confirms that when low protein content ((B)) is used in the gel paste formulation, internal crosslinker should be used as it has a positive effect on the microfibrillar structure development.

### Example 10 (E.10)

Procedures as per E.1 with the following adjustments:

Intermediate step between 3 and 4: Optional component preparation Weight an amount of hydrolysate to reach 5 mg for each mL of hydrogel volume and place in on a weighing boat.
Quickly transfer it to an Eppendorf tube.
Step 5:
after the first mixing of the hydrogel mixture, add the weighted optional component to the hydrogel mixture and mix properly.

Results: With the same composition of E.1 but with the addition of a bioactive protein hydrolysate, in E.10 we assessed the possibility of including bioactive protein-based molecules at the hydrogel level to further boost the differentiation performance. Qualitative assessment of the microfibrillar structure showed that the presence of the protein hydrolysate influences the microfibrillar structure development. In comparison to E.1, in E.10 a lower MF was identified. Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). On the other hand, formulation of Example 10 leads to an increase of differentiation performance of 61.9-folds in comparison to the reference (2D control). These folds of increase for E.10 were much higher than what was observed for E.1.

This example confirms that bioactive protein-based compounds (hydrolysates) can be used to boost the differentiation performance.

**Example 11 (E.11)**

Procedures as per E.1 with the following adjustments:

Intermediate step between 3 and 4: Optional component preparation Weigh an amount of hydrolysate to reach 20 mg for each mL of hydrogel volume and place in on a weighing boat.
Quickly transfer it to an Eppendorf tube.
Step 5:
after the first mixing of the hydrogel mixture, add the weighted optional component to the hydrogel mixture and mix properly.

Results: With the same composition of E.10 but with 4-times higher addition of a bioactive protein hydrolysate, in E.11 we assessed the role of hydrolysate concentration on differentiation performance and microfibrillar structure development. Qualitative assessment of the microfibrillar structure showed that the presence of higher protein hydrolysate does not have a notable influence on the microfibrillar structure development. Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). On the other hand, formulation of Example 11 leads to an increase of differentiation performance of 13.1-folds in comparison to the reference (2D control). These folds of increase for E.11 are much lower than what was observed for E.10, and within the same range of E.1.

This example suggests that bioactive protein-based compounds (hydrolysates) can be used to boost the differentiation performance within a certain limit of concentration.

**Example 12 (E.12)**

Taking the formulation of E.3 as a reference, in this example we applied different differentiation culturing conditions developed in-house to maximise nutritional attributes. Procedures as per E.1 with the following adjustments:

Step 1:

for (1.3) stock solution, weight 3.2 g of Gelatin A on a weight scale;
no enzymatic IX stock needs to be prepared

Step 5:

when adding 500 ul of (1.2) to the tube containing (1.3), we will end up having a volume of 1 ml ((1.3) 8% and (1.2) 2% with 18 million cells per mL);
no addition of enzymatic IX;

Results: With the same composition of E.3, in this example we tested the possibility of producing muscle bundles with high nutritional attributes by employing in-house developed differentiation conditions. Gene expression of MYH7 (gene encoding a myosin heavy chain isoform expressed in skeletal muscle) was used to assess the influence of the microfibrillar structure of the hydrogel fibers (3D cell culture) on differentiation performance. This was evaluated by looking at the expression of MYH7 in comparison to 2D culture under the same culturing conditions (4 days of differentiation, same differentiation media formulation, same cell type). On the other hand, formulation of E.12 leads to an increase of differentiation performance of 44.3-folds in comparison to the reference (2D control). In addition, from the BCA assay, the fraction of extracted protein from the cultivated tissue was roughly 75% of those extracted from a lean muscle tissue biopsy. This example confirms the composition and methods disclosed in this invention, allow the production of cultivated muscle bundles with high nutritional value (comparable protein content of a muscle tissue from a living animal).

LIST OF REFERENCE SIGNS AND ABBREVIATIONS

| BF | bioactivity factor | MF | microfibrillar factor |
|----|--------------------|----|----------------------|
| DF | drawing factor | MSA | microstructuring agent |
| OS | overall score | $S_p$ | pulling speed |
| $S_e$ | extrusion speed | | |

**Claims**

1. Paste for the fabrication of cultivated meat, comprising of consisting of the following components:

(A) at least one polysaccharide crosslinkable to form a gel by the action of divalent or polyvalent cations, light-induced addition reaction or light induced condensation reaction, or a combination thereof, in a concentration in the paste in the range of 0.01-200 g per L of component (D);
(B) at least one protein assembling with the polysaccharide of component (A) via supramolecular or covalent interaction or a combination thereof, in a concentration in the paste in the range of 0.001-500 g per L of component (D);
(C) cells selected from mammalian cells, fish cells, crustaceous cells or a combination thereof, in a concentration in the paste in the range of 0-300 billion cells per L of component (D);
(D) water or a water-based culturing medium;
(E) additives different from (A)-(D), selected from the group consisting of crosslinking kinetic modifier in a concentration in the paste in the range of 0-500 mM, flow modifier in a concentration in the paste in the range of 0-200 g per L of component (D), or a combination thereof.

2. Paste according to claim 1, wherein the at least one polysaccharide of component (A) is naturally crosslinkable and/or chemically modified to promote crosslinking, wherein preferably the chemical modifications include acrylation, methacrylation, epoxidation, allylation, or a combination thereof,

and/or wherein the at least one polysaccharide of component (A) is selected from the group consisting of alginates, pectins, carrageenans, chondroitin sulfate, dermatan sulfate, heparin, heparin sulfate, as well as derivatives thereof and combinations thereof, preferably the polysaccharide component of component (A) is at least one alginate or alginate derivative
and/or wherein the polysaccharide(s) of component (A) is present in a concentration in the paste in the range of 0.1-100 g per L of component (D).

3. Paste according to any of the preceding claims, wherein the at least one protein of component (B) is selected from the group consisting of gelatin, collagen, fibrinogen, fibrin, fibronectin, fibroin, elastin, laminin, cardosin A, albumin, globulin, casein from milk as well as albumins, globulins, prolamins, glutenins, isolated from plant proteins, each in natural, synthetic, or recombinant form, as well as combinations or fractions thereof,

and/or wherein the at least one protein of component (B) takes the form of a native protein, denatured protein, protein hydrolysate, or a combination thereof,
and/or wherein the at least one protein of component (B) is bioactive with the ability to modulate a function and/or characteristic of the cells of component (C), in particular to promote cell attachment by providing integrin-binding motifs;
and/or wherein the at least one protein of component (B) is selected from the group consisting of gelatin, collagen, fibrinogen, fibrin, fibronectin, fibroin, elastin, laminin, basic albumins from plant, preferably napin, wherein preferably the protein component (B) is gelatine, more preferably gelatine, type A,
and/or wherein the at least one protein of component (B) is chemically modified to promote crosslinking, wherein preferably the chemical modifications include acrylation, methacrylation, epoxidation, allylation, or a combination thereof,
and/or wherein the at least one protein of component (B) is crosslinked, preferably by transglutaminase, peroxidase, laccase, tyrosinase, lysyl oxidase, glutaraldehyde, genipin, citric acid, photopolymerization or a combination thereof, preferably by transglutaminase is used, and wherein if transglutaminase is used as crosslinker, it is comprised in an amount of 0-50 U per g of protein, more preferably 0.1-10 U per g of protein, and/or wherein the protein(s) of component (B) is present in a concentration in the paste in the range of 0.1-250 g per L of component (D).

4. Paste according to any of the preceding claims, wherein the cells of component (C) are selected from the group consisting of embryonic stem cells, adult stem cells, induced stem cells, fibroblasts, fibrocytes chondrocytes or a combination thereof, and/or wherein the cells of component (C) are present in a concentration in the paste in the range of 5-100 billion cells per L of component (D).

5. Paste according to any of the preceding claims, wherein the additives of component (E) include at least one crosslinking kinetic modifier to slow down the gelation kinetics of the crosslinkable polysaccharide by sequestering divalent or polyvalent cations,

and/or wherein the additives of component (E) include at least one food-compatible compound selected from the group consisting of disodium phosphate, dipotassium phosphate, magnesium phosphate, ethylenediaminetetraacetic acid, ethylenediaminetetraacetic salts, or a combination thereof, and/or wherein the additives of component (E) are present in a concentration 1-100 mM, and/or wherein the additives of component (E) include at least one flow modifier to impart a shear-thinning behaviour to the gel past so that the higher viscosity in resting condition mitigate cell sedimentation, whereas the lower viscosity during ejection allows processability, wherein preferably said flow modifiers are edible fillers in the form of micro/nanofibers, preferably insoluble in culturing conditions, and are preferably composed of protein and/or polysaccharide different from the other components of the paste, wherein such edible filler is preferably present at a concentration of 0-200g per L of paste, and wherein further preferably edible polymers as flow modifier can be selected among gums, including gellan gums, guar gums, xanthan gums), PEGs, or combinations thereof, and wherein preferably edible polymers are present at a concentration of 0-50g per L of gel paste.

6. Paste according to any of the preceding claims, wherein it further comprises a micro-structuring agent, for unfolding of protein structure, preferably selected from the group consisting of bicarbonate, including sodium bicarbonate, potassium bicarbonate, ammonium bicarbonate, magnesium bicarbonate, or a combination thereof,

wherein preferably the micro-structuring agent is added to the paste in an amount to lead to at a concentration ranging from 0.1-300 g per L of paste, preferably, of 1-200 g per L of paste, wherein the micro-structuring agent is preferably added in powder form.

7. Method for the manufacturing of a microfibril structure for the fabrication of cultivated meat, wherein

(a) a paste according to any of the preceding claims is supplemented with a food compatible micro-structuring agent for unfolding of protein structure
(b) the mixture is extruded through and ejection nozzle with an ejection speed ($S_e$) under the generation of a speed gradient downstream of said nozzle with a pulling speed ($S_p$) larger than the ejection speed ($S_e$) to form a drawn paste, and
(c) while drawing or after drawing the drawn paste is immersed in a hardening bath, preferably a hardening bath comprising divalent or polyvalent cations or an acidic bath.

8. Method according to claim 7, wherein the micro-structuring agent used in step (a) is selected from the group consisting of bicarbonate, including sodium bicarbonate, potassium bicarbonate, ammonium bicarbonate, magnesium bicarbonate, or a combination thereof,

and/or wherein the micro-structuring agent is added to the paste in an amount to lead to at a concentration ranging from 0.1-300 g per L of paste, preferably, of 1-200 g per L of paste, and/or wherein the micro-structuring agent is added in powder form.

9. Method according to any of the preceding claims 6 or 8, wherein the extrusion in step (b) takes place with a drawing factor, defined as the ratio of the ejection speed ($S_e$) to the pulling speed ($S_p$), of at least 1.1, preferably at least 1.5, and/or wherein fiber for the drawing is blocked on a clamping platform allowing to apply the drawing factor by moving at least one of the ejection nozzle or the clamping platform at a pulling speed ($S_p$), wherein the movement of the camping platform can be manual or automated and wherein the movement of the camping platform can be linear or rotative, or a combination thereof.

10. Method according to any of the preceding claims 7 - 9, wherein the hardening bath of step (c) comprises $Ca^{2+}$, $Mg^{2+}$, $Fe^{2+}$ and $Fe^{3+}$ or a combination thereof in water, preferably at a concentration in the range of 1-500 mM, more preferably between 20-100 mM,
and/or wherein the hardening bath of step (c) is buffered at a pH ranging from 6 to 8, preferably by using HEPES buffer, wherein further preferably the HEPES buffer is used in a concentration comprised between 1-100mM.

11. Method according to any of the preceding claims 7-10, wherein in step (b) the paste is extruded from a nozzle, preferably a nozzle with a circular geometry at the ejection point,

and/or wherein in step (b) the paste is extruded from a nozzle with a diameter in direction perpendicular to the ejection direction with the largest extension in the range of between 10 to 5000 $\mu$m, more preferably 100-1000 $\mu$m,
and/or wherein in step (b) the pulling rate ($S_p$), relative to the nozzle, is in the range of 0.01-100 m/min, more preferably between 0.1-10 m/min.

12. Method according to any of the preceding claims 7-11, wherein either the hardening bath comprises a protein cross-linking agent or subsequent to step (c) the fibres are immersed in a protein cross-linking bath with such a cross-linking agent, wherein preferably the cross-linking agent is selected from the group consisting of transglutaminase, peroxidase, laccase, tyrosinase, lysyl oxidase, glutaraldehyde, genipin, citric acid, or a combination thereof, preferably transglutaminase, and wherein if transglutaminase is used, it is comprised in an amount of 1-2000 U per mL of crosslinking bath, and wherein further preferably cross-linking is performed at a temperature in the range of 20-45°C, preferably for a time span in the range of 10-120 min, more preferably 30-90 min.

13. Method according to any of the preceding claims 7-12, wherein the resulting fibres, preferably consisting of fibrils having a diameter in the range of 0.1-50 $\mu$m, preferably aligned in the same axis $\pm$ 40° with respect of the fiber axis, are converted into muscle tissue by culturing them in differentiation media designed for the used cell type, wherein if the fibres have been produced from a paste without cells, before culturing the fibres are seeded with cells, and wherein preferably the fibers, preferably in the form of bundles, are cross-linked together to obtain a solid edible structure, wherein cross-linking of the fibres can be effected by one or several cross-linking agents selected from the group consisting of transglutaminase, peroxidase, laccase, tyrosinase, lysyl oxidase, glutaraldehyde, genipin, citric acid, or a combination thereof, preferably transglutaminase.

14. Method according to claim 13, wherein the resulting muscle tissue is mixed with further constituent to form a consumer cultured meat product.

15. Cultured meat based on a paste according to any of the preceding claims 1-6, and/or obtained using a method according to any of the preceding claims 7-14.

FIG. 1

FIG. 2

1. Microfibrillar structure arises from the unique formulation composition and fabrication method

Gradient of speed along this axis

Hydrogel fibrils

Cells trapped in the fibrillar structure

2. Cells are trapped in small fibrils

3. Microfibrillar structure provides topological cues allowing cells to spread along the fiber axes

FIG. 3a

A **Cell paste ejector**

Polysaccharide component

Protein component

Microstructuring agent

**MSA**

Paste ejection (1)

Paste ejection — Non-gelled paste

Partially gelled paste

Gelled paste

Gel pulling (2)

B

Gelled paste

Resulting fibrillar structure

C **Hydrogel fiber consisting of aligned microfibrils**

**Pulling axis**

**FIG. 3b**

FIG. 4

Cell paste ejector

$S_e$

Paste ejection

$S_p$

Volumetric blocking

Rotative clamping platform

Platform rotation

B

A

Cell paste ejector

$S_e$

Paste ejection

$S_p$

Volumetric blocking

Planar clamping platform

Platform translation

Legend

Non-gelled paste

Partially gelled paste

Gelled paste

$S_e$  Ejection speed

$S_p$  Pulling speed

BF = 0

BF = 0.5

BF = 1

MF = 0

MF = 0.5

MF = 1

FIG. 5

FIG. 6

430 µm

Gene expression

7000

5250

3500

1750

0

Folds of increase of MYH7 in comparison to an undifferentiated reference

2D differentiated cell culture

Cultivated muscle tissue, E.1

FIG. 7

FIG. 8

430 μm

FIG. 9

430 μm

FIG. 10

430 μm

430 µm

FIG. 11

430 µm

FIG. 12

430 µm

FIG. 13

**FIG. 14**

**FIG. 15**

**FIG. 16**

FIG. 17

FIG. 18

FIG. 19

**FIG. 20**

**FIG. 21**

Gene expression (RT-qPCR)

Protein quantification (BCA)

FIG. 22

# EP 4 252 549 A1

Europäisches
Patentamt

European
Patent Office

Office européen
des brevets

## EUROPEAN SEARCH REPORT

Application Number

EP 22 16 4793

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/345643 A1 (SCIONTI GIUSEPPE [ES]) 11 November 2021 (2021-11-11) * example 4 * | 1-6 | INV. A23L13/00 A23L17/00 C12N5/00 |
| Y | ZIDARIC TANJA ET AL: "Cultured Meat: Meat Industry Hand in Hand with Biomedical Production Methods", FOOD ENGINEERING REVIEWS, SPRINGER, US, vol. 12, no. 4, 26 August 2020 (2020-08-26), pages 498-519, XP037289819, ISSN: 1866-7910, DOI: 10.1007/S12393-020-09253-W [retrieved on 2020-08-26] | 14,15 | C12N5/077 A23J3/22 |
| A | * page 503, column 2, paragraph 2 - page 505, column 2, paragraph 1 * | 1-13 | |
| A | CASSIMJEE HENNA ET AL: "Proteosaccharide combinations for tissue engineering applications", CARBOHYDRATE POLYMERS, APPLIED SCIENCE PUBLISHERS , LTD BARKING, GB, vol. 235, February 2020 (2020-02), XP086068299, ISSN: 0144-8617, DOI: 10.1016/J.CARBPOL.2020.115932 [retrieved on 2020-02-01] * page 3, column 2, paragraph 3 - page 5, column 1, paragraph 1 * * page 7, column 1, paragraph 4 - page 11, column 1, paragraph 1 * | 1-15 | TECHNICAL FIELDS SEARCHED (IPC) A23L C12N A23J |

-/--

The present search report has been drawn up for all claims

| Place of search | Date of completion of the search | Examiner |
|---|---|---|
| Munich | 15 September 2022 | Schlegel, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 22 16 4793

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DISTLER THOMAS ET AL: "3D printed oxidized alginate-gelatin bioink provides guidance for C2C12 muscle precursor cell orientation and differentiation via shear stress during bioprinting", BIOFABRICATION, vol. 12, no. 4, 7 July 2020 (2020-07-07), page 045005, XP55961000, UK ISSN: 1758-5082, DOI: 10.1088/1758-5090/ab98e4 * page 2, column 2, paragraph 3 - page 4, column 2, paragraph 1 * * page 5, column 2, paragraph 3 - page 10, column 1, paragraph 1 * * figures 1,4 * | 7-15 | |
| A | FARIBA DEHGHANI ET AL: "Engineering porous scaffolds using gas-based techniques", CURRENT OPINION IN BIOTECHNOLOGY, vol. 22, no. 5, 3 May 2011 (2011-05-03), pages 661-666, XP028303034, ISSN: 0958-1669, DOI: 10.1016/J.COPBIO.2011.04.005 [retrieved on 2011-04-14] * page 661 - page 662, column 2, paragraph 1 * * page 664, column 1, paragraph 2 - column 2 * | 7-15 | TECHNICAL FIELDS SEARCHED (IPC) |
| A | US 6 511 650 B1 (EISELT PETRA [US] ET AL) 28 January 2003 (2003-01-28) * the whole document * | 7-15 | |
| Y | EP 2 547 810 B1 (AMSILK GMBH [DE]) 6 March 2019 (2019-03-06) * paragraphs [0121] - [0123], [0141] * | 7-15 | |

The present search report has been drawn up for all claims

| Place of search | Date of completion of the search | Examiner |
|---|---|---|
| Munich | 15 September 2022 | Schlegel, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 22 16 4793

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2022

| Patent document cited in search report | | Publication date | Patent family member(s) | | Publication date |
|---|---|---|---|---|---|
| US 2021345643 | A1 | 11-11-2021 | AR 115915 | A1 | 10-03-2021 |
| | | | AU 2019316690 | A1 | 04-02-2021 |
| | | | BR 112021002252 | A2 | 04-05-2021 |
| | | | CA 3107569 | A1 | 13-02-2020 |
| | | | CL 2021000275 | A1 | 19-07-2021 |
| | | | CN 112839522 | A | 25-05-2021 |
| | | | CO 2021002711 | A2 | 20-05-2021 |
| | | | EP 3833194 | A1 | 16-06-2021 |
| | | | IL 280604 | A | 25-03-2021 |
| | | | JP 2021533749 | A | 09-12-2021 |
| | | | KR 20210052438 | A | 10-05-2021 |
| | | | US 2021345643 | A1 | 11-11-2021 |
| | | | WO 2020030628 | A1 | 13-02-2020 |
| US 6511650 | B1 | 28-01-2003 | AU 776009 | B2 | 26-08-2004 |
| | | | CA 2364570 | A1 | 19-10-2000 |
| | | | EP 1169378 | A1 | 09-01-2002 |
| | | | JP 2002542326 | A | 10-12-2002 |
| | | | US 6511650 | B1 | 28-01-2003 |
| | | | WO 0061668 | A1 | 19-10-2000 |
| EP 2547810 | B1 | 06-03-2019 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020160533 A
- US 7622299 B
- US 8273373 B
- WO 2021158105 A
- WO 2006101453 A1

**Non-patent literature cited in the description**

- **MACQUEEN.** Muscle tissue engineering in fibrous gelatin: implications for meat analogs. *npj Sci Food,* 2019, vol. 3, 20
- **KANG et al.** Engineered whole cut meat-like tissue by the assembly of cell fibers using tendon-gel integrated bioprinting. *NATURE COMMUNICATIONS,* 2021, vol. 12, 5059
- **DISTLER et al.** 3D printed oxidized alginate-gelatin bioink provides guidance for C2C12 muscle precursor cell orientation and differentiation via shear stress during bioprinting. *Biofabrication,* 2020, vol. 12, 045005
- **KESSEL et al.** 3D Bioprinting of Macroporous Materials Based on Entangled Hydrogel Microstrands. *Adv. Sci.,* 2020, vol. 7, 2001419
- **ZHANG et al.** Creating polymer hydrogel microfibres with internal alignment via electrical and mechanical stretching. *Biomaterials,* March 2014, vol. 35 (10), 3243-3251